# EUROPÄISCHE PATENTANMELDUNG

(11) **EP 2 322 639 A1**
(43) Veröffentlichungstag der Anmeldung: **18.05.2011**
(21) Anmeldenummer: 10193564.1
(22) Anmeldetag: 09.03.2007
(51) Int. Cl.: C12P 7/06, C12P 7/08, C12M 1/40, C12M 1/42

(54) **Verfahren zur Herstellung von Ethanol aus lactosehaltigen Stoffen**

(30) Priorität: 09.03.2006 DE 102006010985
(62) Teilanmeldung aus: 07722009.3
(71) Anmelder: Georg Fritzmeier GmbH + Co. KG, 85655 Grosshelfendorf (DE)
(72) Erfinder:
(74) Vertreter: Winter, Brandl, Fürniss, Hübner, Röss, Kaiser, Polte - Partnerschaft

(57) **Zusammenfassung**

Offenbart ist ein Verfahren zur Herstellung von Ethanol aus saccharidhaltigen Stoffen, insbesondere Molke mit den Schritten:
- Entfernen von Hemmstoffen, insbesondere Kupfer, aus dem Stoff;
- Umwandeln von in dem Stoff enthaltener Laktose in Glukose;
- Zugeben von Mikroorganismen, insbesondere Hefe und/oder Bakterien, zur Herstellung von Ethanol aus Glukose.

## Beschreibung

Vorliegende Erfindung betrifft ein Verfahren zur Herstellung von Ethanol, insbesondere aus Molke.

Aus dem Stand der Technik ist bekannt, dass Ethanol aus saccharidhaltigen Stoffen, insbesondere Zucker bzw. Glukose, gewonnen werden kann. Dabei sind Getreide, Zuckerrüben, Kartoffeln und Molke als Glukoselieferanten bekannt, die sich zur Ethanolherstellung eignen. Aber auch andere saccharidhaltige Lebensmittel und vor allem Lebensmittelrückstände können vergoren werden.

Problematisch ist dabei jedoch, dass zum einen gerade in Lebensmittelrückständen aufgrund von beginnender Zersetzung, hervorgerufen beispielsweise durch Schimmelpilze oder ähnliches, gärhemmende Stoffe befinden, die eine Ethanolherstellung aus diesen Produkten behindern und deshalb unrentabel machen. Auf der anderen Seite ist gerade die Herstellung von Ethanol aus Molke bis jetzt noch nicht wirtschaftlich, da die aus der Molke gewonnene Ethanolausbeute für eine kommerzielle Auswertung nicht ausreicht.

Aufgabe vorliegender Erfindung ist es deshalb, ein Verfahren bereitzustellen, mit dem gärhemmende Stoffe leicht entfernt werden können und das dadurch eine möglichst einfache und kostengünstige Ethanolherstellung, insbesondere aus der Molke, gewährleistet.

Diese Aufgabe wird durch ein Verfahren zur Herstellung von Ethanol gemäß Patentanspruch 1 gelöst.

Vorliegende Erfindung basiert auf der Erkenntnis, dass die geringe Ethanolausbeute bei der Ethanolherstellung aus saccharidhaltigen Lebensmitteln bzw. Lebensmittelrückständen, wie beispielsweise Molke, auf zwei Probleme zurückzuführen ist:

Zum einen sind bei der Verwendung von Molke als Lebensmittelrückstand, d.h. als Abfallprodukt von Käsereien Hemmstoffe in der Molke vorhanden, die insgesamt die Ethanolherstellung stark einschränken. Zu diesen Hemmstoffen gehört insbesondere Kupfer, das eine Vergärung von Molke zu Ethanol fast unmöglich macht, aber auch Schimmelpilze, die sich durch eine nicht sofortige Weiterverarbeitung der Molke ansiedeln. Solche Hemmstoffe sind auch bei anderen saccharidhaltigen vergärbaren Lebensmitteln, wie beispielsweise Trauben, oder Gerste vorhanden und müssen für eine Ethanolherstellung aus dem zu vergärenden Fluid entfernt werden.

In einem Ausführungsbeispiel wird eine mikrobiotische Mischung nicht dem Fluid direkt zugegeben, sondern die Stoffe, aus denen das Fluid hergestellt wird, werden mit der mikrobiotischen Mischung vorbehandelt. Dies ist insbesondere bei der Herstellung von Wein oder Bier vorteilhaft, bei denen die Trauben oder Äpfel bzw. die Braugerste vor Ansetzen von Most bzw. Maische mit der mikrobiotischen Mischung behandelt werden. Dadurch können gärhemmende Stoffe, wie beispielsweise Mehltau, bereits vor dem Gärprozess entfernt werden und können auch Most bzw. Maische nicht kontaminieren.

Versuche haben gezeigt, dass zudem durch die Zugabe der mikrobiotischen Mischung zu Most, vor allem aber auch bei der Vorbehandlung von Trauben mit der mikrobiotischen Mischung, der darauf folgende Gärverlauf deutlich beschleunigt werden kann, wobei zudem ein rascher Zuckerabbau und ein rasche Temperaturanstieg bei der Gärung beobachtet wurde. Zudem konnte durch die Zugabe der mikrobiotischen Mischung verhindert werden, dass das Gärprodukt muffig oder schlecht roch.

Das andere, insbesondere bei der Ethanolherstellung aus Molke bestehende, Problem liegt darin, dass die in der Molke enthaltene Glukose nicht frei verfügbar ist. Man kann also nicht einfach nur der Molke Hefe für einen Gärprozess zugeben, sondern die Glukose muss erst aus der Laktose gewonnen werden. Laktose ist meist aber nur zu einem Prozentsatz von 30 - 40% in der Molke enthalten und besteht darüber hinaus nicht zu 100% aus Glukose, sondern auch aus Galaktose. Galaktose ist zwar auch ein Monosaccharid, kann aber nicht zu Ethanol vergärt werden, wodurch die Ethanolausbeute weiter reduziert wird.

Erfindungsgemäß wird deshalb ein Verfahren vorgeschlagen, bei dem in einem ersten Schritt solche Hemmstoffe, wie beispielsweise Kupfer, entfernt werden. In einem sich anschließenden Schritt wird die vorhandene Laktose in Glukose und Galaktose aufgespalten, wobei die Galaktose nicht wie ein Abfallprodukt behandelt wird, sondern wird, wie ein weiteres besonders bevorzugtes Ausführungsbeispiel zeigt, mit Hilfe eines bestimmten Hefestamms, den sogenannten Kluyveromyces, in Glukose umgewandelt.

Erst dann wird in einem dritten Schritt ein Gärmittel, insbesondere Hefe oder Bakterien, zugegeben, um die jetzt vergleichsweise hohe Konzentration an Glukose in Ethanol umzuwandeln.

Da wie oben beschrieben insbesondere Kupfer als Ethanolherstellungshemmstoff identifiziert wurde, wird Kupfer in einem besonders bevorzugten Ausführungsbeispiel mittels Elektrolyse aus dem Stoff entfernt.

Eine weitere Einschränkung ist, dass bei der Gärung zu Ethanol auf den Prozentsatz von Ethanol in der Gärlösung geachtet werden muss, da ein zu hoher Alkoholgehalt den Gärprozess negativ beeinflusst. Deshalb wird, wie ein weiteres vorteilhaftes Ausführungsbeispiel zeigt, der Alkoholgehalt auf einem bestimmten Niveau, vorzugsweise unter 12%, gehalten. Dazu kann vorzugsweise überschüssiges Ethanol aus der Gärlösung mittels Membranfilterung abgeführt werden.

Besonders vorteilhaft ist es, wenn das erfindungsgemäße Verfahren in einem Bioreaktor stattfindet. Dabei können die drei Hauptschritte des Verfahrens hintereinander in dem gleichen Bioreaktor ausgeführt werden. Dazu muss jedoch zwischen den einzelnen Schritten der Bioreaktor für den nächsten Schritt vorbereitet werden.

Da dies umständlich ist, kann, wie ein weiteres bevorzugtes Ausführungsbeispiel zeigt, das Verfahren auch in vorzugsweise drei hintereinander angeordneten Bioreaktoren stattfinden, wobei ein Umrüsten der einzelnen Bioreaktoren aufgrund der ihnen fest zugewiesenen Verfahrensschritte entfällt.

Dabei ist ein erster Bioreaktor vorzugsweise mit einer Elektrolyseeinrichtung ausgestattet, so dass eine Entfernung von Kupfer aus der Molke ermöglicht wird. Dazu kann auf eine spezielle Ausstattung des Bioreaktors zurückgegriffen werden. Ein besonders bevorzugter Bioreaktor besteht nämlich aus einem beschichteten Behälter und einem beschichteten Füllkörper, wobei die Beschichtungen so gewählt sind, dass beim Anlegen eines elektrischen Feldes der Bioreaktor selbst als Elektrolysevorrichtung wirkt. Besonders vorteilhaft haben sich dabei eine photokatalytische Beschichtung des Bioreaktors und eine Aktivkohlebeschichtung des Füllkörpers erwiesen. Beim Anlegen des elektrischen Feldes dient dann der Füllkörper als Anode und die Kupferionen setzten sich an der Aktivkohleschicht ab.

In diesen ersten Bioreaktor wird beispielsweise Molke aus einer Käserei eingeleitet und das störende Kupfer daraus entfernt. Ist die Molke stark belastet, beispielsweise da sie bereits eingelagert war, kann hier der Molke auch die mikrobiotische Mischung zugegeben werden. Ist die Molke nur wenig belastet oder frisch, kann der Molke auch vor dem Einleiten in den ersten Bioreaktor Hopfen zugegeben werden, vorzugsweise 100g Hopfen/hl Molke, so dass zum einen eine bereits vorhandene Belastung gemindert oder zumindest gebremst wird, zum anderen sichergestellt werden kann, dass eine solche Belastung gar nicht erst auftritt.

Die so vorbereitete Molke wird dann in einen zweiten Bioreaktor überführt, in dem die in der Molke enthaltene Laktose mittels Laktase in Glukose und Galaktose aufgespalten wird. Dazu können des weiteren Kluyveromyces-Hefen in dem zweiten Bioreaktor vorgesehen sein, die zum einen ebenfalls eine Aufspaltung von Laktose in Glukose und Galaktose bewirken, zum anderen aber auch dazu fähig sind, Galaktose in Glukose umzuwandeln. In einem dritten Bioreaktor, in den die Glukosemischung eingeleitet wird, sind Mikroorganismen, insbesondere Hefen oder Gärbakterien, vorhanden, die eine Umwandlung von Glukose mittels alkoholischer Gärung in Ethanol bewirken. Das so produzierte Ethanol wird dann vorzugsweise mittels einer Membran aus der Glukosemischung abgeschieden.

Weitere bevorzugte Ausführungsbeispiele und Vorteile sind in den Unteransprüchen definiert.

Im Folgenden werden besonders bevorzugte Ausführungsbeispiele der Erfindung an Hand von Figuren noch näher erklärt. Dabei sind die Figuren rein exemplarisch und sollen nicht dazu verwendet werden, den Rahmen der Patentansprüche auf die gezeigten Ausführungsbeispiele einzuschränken.

Es zeigen:
Figur 1: eine schematische Zeichnung eines bevorzugten Ausführungsbeispiels vorliegender Erfindung, bei dem drei Bioreaktoren in Reihe hintereinander geschaltet sind, um das erfindungsgemäße Verfahren zur Ethanolherstellung aus Molke auszuführen;
Figur 2: eine schematische Zeichnung eines bevorzugten Ausführungsbeispiels eines Bioreaktors für das erfindungsgemäße Verfahren; und
Figur 3: eine schematische Zeichnung eines weiteren Ausführungsbeispiels vorliegender Erfindung, um das erfindungsgemäße Verfahren zur Ethanolherstellung aus Molke auszuführen.

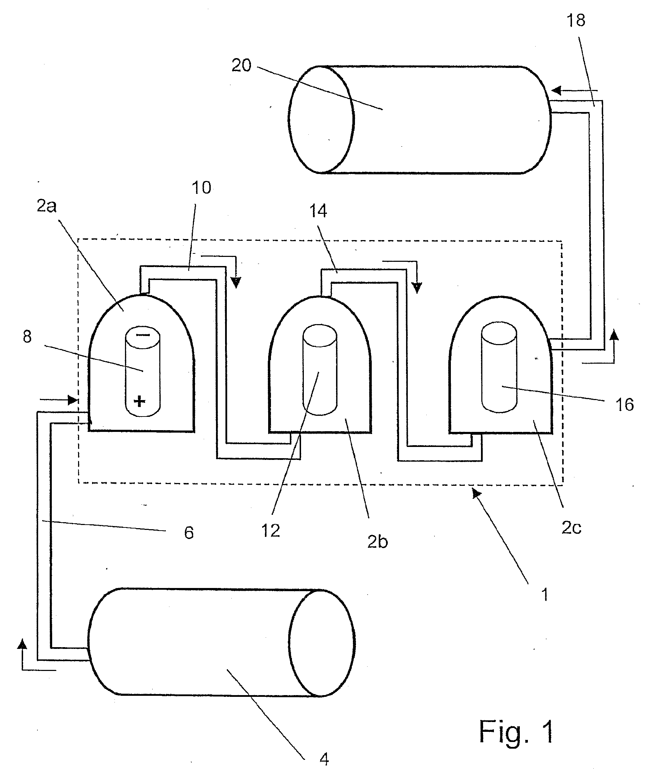

Figur 1 zeigt ein bevorzugtes Ausführungsbeispiel für eine Anordnung 1 von Bioreaktoren 2a - c für die Durchführung des erfindungsgemäßen Verfahrens.

Um Ethanol aus Molke herzustellen, wird Molke, die sonst als Abfallprodukt von Käsereien entsteht, weiterbehandelt. Dazu wird die Molke aus einem Lager- oder Anliefertank 4 über eine erste Leitung 6 in die Anordnung 1 von Bioreaktoren 2a - c überführt. Es ist aber auch möglich, direkt an der Käserei selbst eine solche Anlage oder zumindest einen Bioreaktor für die Ethanolherstellung vorzusehen. Dann könnte die Molke direkt über die Leitung 6 aus der Käserei in die Anordnung 1 geleitet werden, ohne dass ein Tank 4 erforderlich wäre.

Um eine bakteriologische Belastung der Molke zu verhindern oder eine solche zu verringern, kann der Molke in dem Tank 4 Hopfen, vorzugsweise 100 g Hopfen/hl Molke zugegeben werden. Hopfen wirkt mykotisch selektiv und unterdrückt die grampositiven Bakterien, wodurch sich in der Molke enthaltene Fremdkeime nicht weiter vermehren können. Dies bewirkt zudem, dass vor allem die zuckerverstoffwechselnden Bakterien, die die Ethanolausbeute aufgrund der Zuckergehaltreduzierung verringern, in ihrer Vermehrung gehindert werden. Dabei kann Hopfen als Gesamtstoff zugegeben werden, es ist jedoch auch möglich, nur die für die bakteriostatische Wirkung des Hopfens nötigen β-Säuren des Hopfens als Hopfenauszug der Molke zuzugeben.

Ist die Molke bereits stark mit Fremdkeimen belastet, ist es zudem vorteilhaft, der Molke eine mikrobiotische Mischung aus photosynthetisch arbeitenden Mikroorganismen und Licht emittierenden Mikroorganismen zuzugeben. Diese mikrobiotische Mischung ist auch dazu geeignet, in der Molke vorhandene Schimmelpilze zu beseitigen.

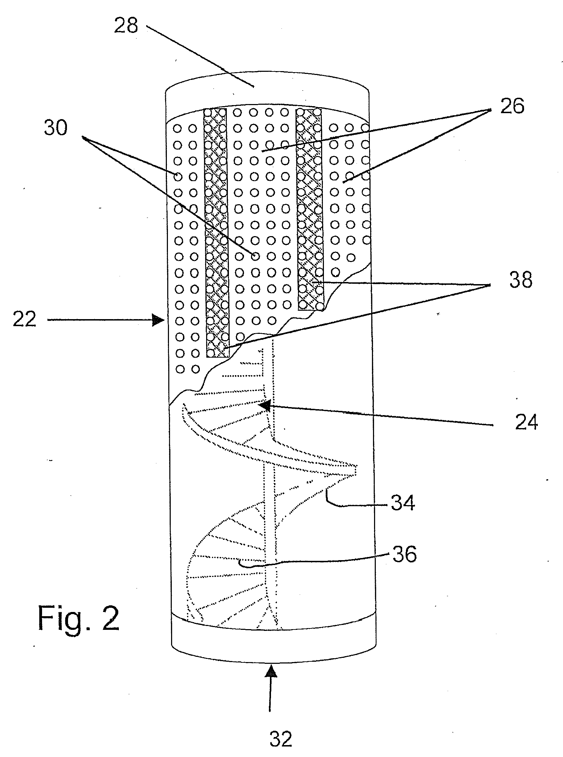

In einem ersten Bioreaktor 2a wird die Molke von Hemmstoffen für die Ethanolherstellung getrennt. Dies kann, insbesondere beim Vorhandensein von Kupfer, über Elektrolyse erfolgen. Zudem kann in dem Bioreaktor 2a die Zugabe der mikrobiotischen Mischung erfolgen. Für die Elektrolyse ist eine Elektrolysevorrichtung 8 vorgesehen, an der die Kupferionen an der Anode abgeschieden werden. Die Elektrolysevorrichtung kann auch als beschichteter Bioreaktor mit Füllkörper ausgebildet sein, bei dem sich durch eine abwechselnde Beschichtung aus Diamant und beispielsweise Titandioxid und einer Aktivkohlebeschichtung des Füllkörpers ein elektrisches Feld bildet. Der Füllkörper kann die Form einer Spindel aufweisen, die wiederum die Beschichtung aus Aktivkohle besitzt. Eine schematische Darstellung eines solchen Bioreaktors mit Füllkörper wird detailliert in Figur 2 beschrieben.

Da dieses elektrische Feld jedoch recht schwach ist, sollte für ein gutes Ergebnis eine externe Stromquelle für die Elektrolyse verwendet werden. Dazu wird ein schwacher Strom zwischen 400 und 600 mA, bzw. 0,3 - 2,6 V, vorzugsweise 1,9 V, angelegt, und der Füllkörper als Anode verwendet.

Da die Kupferionen nicht frei in der Molke vorhanden sind, sondern an die schon in der Molke enthaltenen für die Aufspaltung von Laktose in Glukose und Galaktose verantwortlichen Laktaseenzyme gebunden sind, ist es sinnvoll diese Enzyme wieder zurück zu gewinnen, um nicht unnötig viele Enzyme im darauf folgenden Verfahrensschritt der Laktoseaufspaltung einsetzen zu müssen. Dazu wird der angelegte Strom abgeschaltet und eine Spannungsumkehr initialisiert, worauf sich die Enzyme von dem Füllkörper lösen, während die Kupferionen noch am Füllkörper bleiben. Wird zur gleichen Zeit die so behandelte Molke aus dem ersten Bioreaktor 2a über eine weitere Leitung 10 entfernt, befinden sich in der Molke die Laktaseenzyme, aber keine bzw. verschwindend wenige Kupferionen.

Kommt die Molke direkt aus der Käserei, hat sie meist eine Temperatur von 45°C - 55°C. Für die Elektrolyse spielt die Temperatur keine Rolle, so dass der Bioreaktor 2a nicht mit einer Temperaturkontrolleinheit ausgerüstet werden muss.

Zusätzlich kann die Molke im Bioreaktor 2a noch aufkonzentriert werden. D.h., überschüssiges Wasser wird aus der Molke entfernt, so dass eine hohe Laktosekonzentration erreicht wird.

Ist die Molke aufbereitet, wird sie über die zweite Leitung 10 in einen weiteren Bioreaktor 2b geleitet. Dort findet eine Aufspaltung von Laktose in ihre beiden Bestandteile Glukose und Galaktose statt. Vorzugsweise geschieht dies bei einer Temperatur von 30°C - 35°C. Ist die Molke nicht schon auf diese Temperatur abgekühlt, weil sie beispielsweise direkt aus der Käserei kommt, oder noch zu kalt, muss dem Bioreaktor 2b ein Kühl- bzw. Heizaggregat (hier nicht dargestellt) vorgeschaltet oder in den Bioreaktor integriert sein, das die Molke vor der Aufspaltung der Laktose auf die gewünschte Temperatur bringt. Weiterhin kann auch in dem Bioreaktor selbst ein Temperaturfühler (hier nicht dargestellt) angeordnet sein, der die Reaktionstemperatur ständig überwacht.

Für die Laktoseaufspaltung selbst sind Enzyme - die sogenannte Laktase - zuständig, die vorteilhafterweise in einem in dem Bioreaktor aufgenommenen, in einem Behälter 12 beinhalteten Füllkörper immobilisiert sind. Ein genauer Aufbau des Bioreaktors wird in Figur 2 beschrieben. Der Füllkörper vergrößert zum einen die Reaktionsfläche und zum anderen stellt er eine einfache Möglichkeit bereit, auch bei der Verwendung von nur einem einzigen Bioreaktor durch einfachen Austausch der Füllkörper von dem Bioreaktor für Schritt B - also der Aufspaltung von Laktose - auf einen Bioreaktor für Schritt C - Ethanolherstellung - umzurüsten.

Als Enzym wird Laktase verwendet, das vorteilhafterweise durch Kluyveromyces-Hefen bereitgestellt wird. Der große Vorteil an der Verwendung von Kluyveromyces-Hefen ist, dass durch die von ihnen bereitgestellte Laktase nicht nur eine Aufspaltung von Laktose in Glukose und Galaktose erreicht wird, sondern auch eine Umwandlung von Galaktose in Glukose möglich ist. Dies ist für die Ethanolherstellung von entscheidendem Vorteil, da dadurch der Glukosegehalt und damit die Menge des Ethanol gesteigert wird.

Vorteilhafterweise werden weniger als 10 mg/l Enzyme, optimalerweise 1 mg/l Enzyme der Molke zugegeben. Während einer vorteilhaften Dauer von 8-10 Stunden, spalten dann die Enzyme Laktose zu Glukose und Galaktose auf. Während weiterer 14 -16 Stunden erfolgt dann auch die Umwandlung von Galaktose in Glukose.

Als Ergebnis erhält man eine relativ hochkonzentrierte Glukosemischung, die zur weiteren Bearbeitung in dem hier dargestellten Ausführungsbeispiel über eine dritte Leitung 14 in einen weiteren Bioreaktor 2c überführt wird. Wie eingangs bereits erwähnt, können auch die Bioreaktoren 2b und 2c integral ausgebildet sein. In diesem würde jetzt ein Austausch der Füllkörper vorgenommen werden, um den Bioreaktor auf den Gärprozess umzurüsten.

Der Bioreaktor 2c ist mit einem Füllkörper 16 ausgestattet, der als für die Gärung verwendete Mikroorganismen Hefe oder Bakterien enthalten kann. Da beide Gärprozesse nicht bei gleichen Temperaturen stattfinden, ist zwar ein gleichzeitiges Vorhandensein der zwei Mikroorganismenarten im Füllkörper möglich, aktiv sind jedoch nur die Mikroorganismen, für die die entsprechende Temperatur angelegt ist. So findet eine alkoholische Gärung mittels Hefen bei einer Temperatur von weniger als 25°C, vorzugsweise zwischen 8°C und 10°C, statt, während die Gärung mit Hilfe von Bakterien, insbesondere Thermoanaerobacter ethanolicus, bei 60°C - 65°C stattfindet. Um die Temperatur genau zu steuern, kann dem Bioreaktor 2c ein Kühl- oder Heizaggregat (hier nicht dargestellt) vorgeschaltet oder in ihn integriert sein. Ebenso kann eine Temperaturmessvorrichtung vorhanden sein, die kontrolliert, ob die Temperatur den Anforderungen entspricht.

Die Gärmikroorganismen sind durch den Füllkörper 14 in dem Bioreaktor 2c immobilisiert, der gleichzeitig eine vergrößerte Reaktionsfläche zur Verfügung stellt.

Da der Gärprozess durch eine zu hohe Alkoholkonzentration gehemmt wird, muss das schon produzierte Ethanol aus dem Bioreaktor 2c entfernt werden. Dies kann vorteilhafterweise über eine hier nicht dargestellte Diffusionsmembran geschehen. Es ist aber auch der Einsatz einer speziellen Destillationsmembran vorstellbar, die jedoch relativ teuer ist. Idealerweise sollte die Alkoholkonzentration 12 % nicht übersteigen.

Das hergestellte Ethanol kann anschließend über eine weitere Leitung 18 einem Lagertank 20 zugeführt werden.

Figur 2 zeigt eine Prinzipdarstellung eines bevorzugten Ausführungsbeispiels des Bioreaktors, der aus einem Behälter 22 und einem Füllkörper 24 besteht. In diesem Ausführungsbeispiel ist der Behälter 22 zylinderförmig ausgebildet, er kann jedoch auch beliebige andere Formen aufweisen. Die Seitenwände des Behälters 22 sind bei dem dargestellten Ausführungsbeispiel aus Edelstahl hergestellt und partiell mit einer photokatalytisch wirkenden Beschichtung 26 versehen. Diese Beschichtung 26 kann an der Innenumfangswand des Behälters 22 und/oder - wie in Figur 2 gezeigt - an der Außenwand 28 ausgebildet sein. Bei dem dargestellten Ausführungsbeispiel ist der Behälter 22 aus V4A-Stahl hergestellt und mit einer Titandioxid-Beschichtung versehen. Anstelle dieses Titandioxids kann auch Indiumzinnoxid oder dergleichen verwendet werden. Die Außenwand 28 des Behälters 22 ist mit einer Vielzahl von Durchbrüchen 30 versehen, so dass die umzuwandelnde Molke ins Innere des Behälters 22 gelangen kann. Diese Durchbrüche 30 können beispielsweise gestanzt sein, wobei es von Vorteil ist, wenn dann die Stanzgrate nach innen vorstehen. Die untere Stirnfläche 32 des Behälters kann verschlossen sein, so dass das Einströmen der Molke in den Behälter 22 im Wesentlichen in Radialrichtung erfolgt. Die obere Stirnfläche kann ebenfalls verschlossen sein. In dem Fall, in dem die obere Fläche oberhalb des Flüssigkeitsspiegels liegt, kann auf ein Verschließen verzichtet werden.

Im Innenraum des Behälters 22 ist ein auswechselbarer Füllkörper 24 aufgenommen, der, wie in der Aufrissdarstellung gezeigt, eine spiralförmige Struktur aufweist. Dieser Füllkörper 24 besteht bei dem dargestellten Ausführungsbeispiel aus einem Träger 34, der beispielsweise ein spiralförmig gewendeltes Edelstahlblech sein kann. Auf diesem hier gezeigten schraubenlinienförmig gewendelten Träger 34 aus Edelstahl ist beidseitig ein Schaummaterial, beispielsweise ein PU-Schaum aufgebracht, der mit Aktivkohle und ggf. Nano-Composite-Material beschichtet oder versetzt ist. Durch den PU-Schaum wird ein Porensystem gebildet, dessen Wandungen mit Aktivkohle beschichtet sind, so dass eine große Stoffaustauschfläche zur Verfügung gestellt wird.

Konkret besteht bei dem dargestellten Ausführungsbeispiel der Träger 34 aus einem zwei bis drei Millimeter starken VA-Gitterkörper, wobei die wendelförmige Struktur durch zwei Gitterflächen gebildet ist, zwischen denen ein halbharter, offenzelliger PU-Schaum mit Aktivkohlebeschichtung eingebracht ist. Die auf der nach unten gerichteten Seite der Wendel angeordneten Gitterstäbe 36 sind mit einer photokatalytischen Oberfläche versehen, die Maschenweite beträgt an diesen nach unten weisenden Großflächen ca. 10 - 12 mm. An den die nach oben weisende Großfläche der Wendel bildenden Gitterstäben ist keine Beschichtung vorgesehen. Die Maschenweite beträgt hier etwa 25 - 30 mm.

Die eingangs genannten Mikroorganismen und Enzyme können zentral über einen Dosierschlauch in das Zentrum des spiralförmigen Füllkörpers 24 eingebracht werden. Es ist jedoch auch möglich, diese Mikroorganismen und Enzyme mit Nano-Composite-Materialien bereits bei der Herstellung des Füllkörpers 24 ins Porensystem einzubringen. Sehr erfolgversprechend waren Versuche, bei denen die Mikroorganismen bzw. Enzyme und Nano-Composite-Materialien in Chitosan gelöst und diese mit den Nano-Composite-Materialien versetzte Mischung dann - beispielsweise durch Tränken - auf den Füllkörper aufgebracht wird, so dass ein kontinuierliches Zuführen von Mikroorganismen bzw. Enzymen entfällt und lediglich in regelmäßigen Abständen ein Austausch des Füllkörpers 24 erforderlich ist.

Der PU-Schaum ist in dem hier dargestellten Ausführungsbeispiel auf der nach unten weisenden Seite der Wendel mit einem gelartigen Material aus Chitosan beschichtet. In diesem Chitosan sind die Nano-Composite-Materialien eingebettet, welche jeweils ein piezoelektrisches Keramik-System aus PZT-Kurzfasern mit photokatalytischen Beschichtungen darstellt. Ferner sind für die Gärung verantwortliche Mikroorganismen bzw. Laktase produzierende Kluyveromyces-Hefen mit eingebettet.

Der Behälter 22 ist sowohl an seiner Innenfläche als auch an seiner Außenfläche mit der photokatalytisch wirksamen Schicht 26 - also beispielsweise dem Titandioxid - beschichtet. Diese Schicht ist an der Innenfläche, d.h. an der dem Füllkörper 34 zugewandten Seite vollständig aufgetragen, während an der Außenfläche das Titandioxid in Form von Streifen 26 aufgebracht ist, zwischen denen Bereiche verbleiben, die mit einer Diamantbeschichtung 38 versehen sind.

Eine derartige Diamantbeschichtung 38 lässt sich synthetisch herstellen, indem Methan und Wasserstoff sowie eine geeignete Trägersubstanz aus beispielsweise Niob, Silizium oder Keramik in einer Vakuumkammer auf Temperaturen bis etwa 2000°C erhitzt werden. Es kommt dann zu einer Reaktion, bei der sich ein Diamantgitter auf der Trägersubstanz ausbildet. Diese Beschichtung 38 wird dann auf der Außenwand 28 des Behälters 22 aufgebracht, so dass mit einer photokatalytisch wirksamen und mit einer Diamantschicht 38 versehene Bereiche nebeneinander liegen. Diese Bereiche 26, 38 verlaufen in Längsrichtung des Behälters 22. Bei dem dargestellten Ausführungsbeispiel entspricht die Breite der Streifen 26 etwa dem Abstand von vier lochförmigen Durchbrüchen 30, während die Breite der Bereiche 38 wesentlich kleiner ist und etwa dem Abstand zwischen zwei benachbarten Durchbrüchen 30 entspricht.

Im Zusammenwirken mit der katalytischen Beschichtung des Behälters 22 und der vorbeschriebenen Beschichtung und der darin vorhandenen Aktivkohle des wendelförmigen Füllkörpers 24 stellt sich ein vergleichsweise starkes elektromagnetisches Feld ein. Die entstehende Potentialdifferenz liegt an den mit der Diamantbeschichtung 38 versehenen Bereichen an, die dann als Diamantelektroden wirken. Diese Spannung kann dafür eingesetzt werden, die in der Molke enthalten Kupferionen an der als Anode wirkenden Elektrode abzuscheiden. Zusätzlich kann auch eine externe Stromquelle vorhanden sein, die die Elektrolyse von Kupfer aktiv unterstützt. Einzelheiten über das durch die Beschichtung entstehende elektromagnetische Feld sind in der älteren Anmeldung DE 103 30 959.4 offenbart, so dass diesbezügliche weitere Erläuterungen entbehrlich sind.

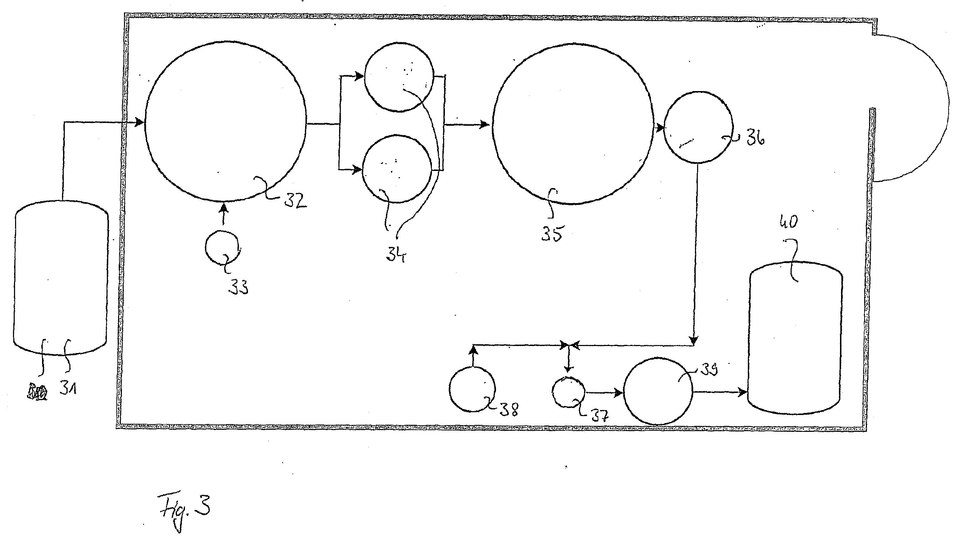

Figur 3 zeigt schematisch ein weiteres Ausführungsbeispiel vorliegender Erfindung, bei dem Ethanol aus Molke gewonnen wird. Aus einem Tank 31 mit Rohmolke, der Hopfen in einem Verhältnis von vorzugsweise 100g/hl zugegeben wird, wird die Molke in einen aeroben Schlaufenreaktor 32 der oben bereits beschriebenen Art eingeleitet. Ist die Molke stark belastet, kann in dem aeroben Schlaufenreaktor der Molke die eingangs beschriebene mikrobiotische Mischung 33 zugegeben werden.

Des Weiteren kann in dem Schlaufenreaktor eine Demineralisierung der Molke stattfinden. Insbesondere Kalium, aber auch Salze, wie beispielsweise NaCl, beeinflussen die Gärung und können diese teilweise stark behindern.

Die so behandelte Molke wird dann in ein Pufferbecken 34 überführt, in dem Molke aus dem Schlaufenreaktor gesammelt wird, um für den weiteren Prozess zur Verfügung zu stehen. Dabei kann Molke aus verschiedensten Molkereien zusammengeführt werden, so dass vorteilhafterweise zudem eine Stabilisierung der Molke erfolgt. Dabei ist es besonders vorteilhaft, wenn in dem Pufferbehälter eine Molke mit einem bestimmten Laktosegehalt vorhanden ist. Dazu kann die eingeleitete Molke entweder verdünnt oder aufkonzentriert werden.

In einem weiteren Behälter 35 wird der pH-Wert der Molke auf den für die nachfolgende Enzymbehandlung nötigen Wert zwischen pH 5 - 7,5, insbesondere pH = 5,8 - 6,3, angehoben. Die Enzymbehandlung und die Aufspaltung von Laktose und Galaktose in Glukose ist bereits oben beschrieben worden.

Nachfolgend werden in einer Membranfiltrationsanlage 36 noch etwaig vorhandene Kleinstteile aus der Molke entfernt, um ein besonders gutes Gärergebnis zu erzielen.

Die so vorbehandelte Molke - eigentlich inzwischen das glukosehaltige Fluid - wird nun in einen Gärbehälter 37 überführt, in dem Gärhefe, die vorzugsweise in einem Hefefermenter 38 vorbehandelt wurde, zugegeben wird. Nach der Gärung erfolgt in einem weiteren Behälter 39 die Hefeabsetzung, um Ethanol in möglichst reiner Form zu erhalten. Daraufhin wird das so erhaltene Ethanol in einen weiteren Behälter 40 überführt, in dem die letzten Reste von Hefe und Zucker zu Ethanol vergoren werden.

## Patentansprüche

1. Verfahren zur Herstellung von Ethanol aus saccharidhaltigen Stoffen, insbesondere Molke **gekennzeichnet durch** folgende Schritte
A. Entfernen von Hemmstoffen, insbesondere Kupfer, aus dem Stoff;
B. Umwandeln von in dem Stoff enthaltener Laktose in Glukose;
C. Zugeben von Mikroorganismen, insbesondere Hefe und/oder Bakterien, zur Herstellung von Ethanol aus Glukose.

2. Verfahren nach Anspruch 1, wobei der Schritt des Umwandelns von Laktose in Glukose umfasst, dass Laktose in Glukose und Galaktose aufgespalten wird, und Galaktose mittels Kluyveromyces - Hefen oder ähnlich wirkenden Organismen in Glukose umgewandelt wird.

3. Verfahren nach Anspruch 1 oder 2, wobei die Hemmstoffe aus dem Stoff mittels Elektrolyse entfernt werden.

4. Verfahren nach einem der Ansprüche 1 bis 3, wobei dem Stoff vor dem Schritt B Hopfen und/oder Hopfenauszüge und/oder eine mikrobiotische Mischung aus photosynthetisch arbeitenden Mikroorganismen und Licht emittierenden Mikroorganismen zugegeben wird.

5. Verfahren nach einem der Ansprüche 1 bis 4, wobei vor dem Schritt C ein Verfahren zum Abbau von gärhemmenden Stoffen durchgeführt wird.

6. Verfahren nach einem der Ansprüche 1 bis 5, wobei bei Schritt C der Ethanolgehalt mittels Diffusion durch eine Ethanol-durchlässige Membran auf einem bestimmten Prozentsatz, insbesondere unter 12 % gehalten wird.

7. Verfahren nach einem der Ansprüche 1 bis 6, wobei der Schritt A bei einer Temperatur zwischen 20°C und 70°C, vorzugsweise bei einer Temperatur zwischen 48°C und 50°C durchgeführt wird.

8. Verfahren nach einem der Ansprüche 1 bis 7, wobei der Schritt B bei einer Temperatur zwischen 10°C und 60°C, vorzugsweise bei einer Temperatur unter 36°C, insbesondere bei einer Temperatur zwischen 28°C und 35°C, durchgeführt wird.

9. Verfahren nach einem der Ansprüche 1 bis 8, wobei der Schritt C bei der Verwendung von Bakterien bei einer Temperatur zwischen 30°C und 80°C, vorzugsweise bei einer Temperatur zwischen 58°C und 68°C durchgeführt wird.

10. Verfahren nach einem der Ansprüche 1 bis 9, wobei der Schritt C bei der Verwendung von Hefen bei einer Temperatur zwischen 0°C und 40°C, vorzugsweise bei einer Temperatur unter 25°C, insbesondere bei einer Temperatur zwischen 5°C und 12°C, durchgeführt wird.

11. Verfahren nach einem der Ansprüche 1 bis 10, wobei die Schritte A, B und/oder C hintereinander in mindestens einem, vorzugsweise in drei in Reihe geschalteten Bioreaktoren (2a-c), durchgeführt werden.
